# EUROPEAN PATENT SPECIFICATION

(11) **EP 1 677 829 B1**
(45) Date of publication and mention of the grant of the patent: **24.12.2014**
(21) Application number: 04768770.2
(22) Date of filing: 07.10.2004
(51) Int. Cl.: A61K 47/48, A61P 7/04

(54) **FIBRINOGEN TARGETTING MICROPARTICLES FOR PROMOTING HAEMOSTASIS**
FIBRINOGEN ANGREIFENDE MIKROTEILCHEN ZUR FÖRDERUNG DER HÄMOSTASE
MICROPARTICULES DE CIBLAGE DU FIBRINOGENE FAVORISANT L'HEMOSTASE

(30) Priority: 07.10.2003 GB 0323378
(43) Date of publication of application: 12.07.2006
(73) Proprietor: Haemostatix Limited, Nottingham NG1 1GF (GB)
(72) Inventor: GOODALL, Alison Helena, Nottingham NG1 1GF (GB); MIDDLETON, Sarah Margaret, Nottingham NG1 1GF (GB)
(74) Representative: Thornton, Neil
(86) International application number: PCT/GB2004/004235
(87) International publication number: WO 2005/035002

(56) References cited:
- EP-A- 0 618 225
- WO-A-98/17319
- WO-A-99/25383
- WO-A-99/42146
- WO-A-2004/045542
- LEVI M ET AL: "FIBRINOGEN-COATED ALBUMIN MICROCAPSULES REDUCE BLEEDING IN SEVERELYTHROMBOCYTOPENIC RABBITS" NATURE MEDICINE, NATURE PUBLISHING, CO, US, vol. 5, no. 1, January 1999 (1999-01), pages 107-111, XP002930943 ISSN: 1078-8956
- DAVIES A R ET AL: "Interactions of platelets with SynthocytesTM, a novel platelet substitute" PLATELETS (ABINGDON), vol. 13, no. 4, June 2002 (2002-06), pages 197-205, XP009043550 ISSN: 0953-7104 cited in the application
- KUYAS C ET AL: "Isolation of human fibrinogen and its derivatives by affinity chromatography on Gly-Pro-Arg-Pro-Lys-Fractogel." THROMBOSIS AND HAEMOSTASIS. 28 JUN 1990, vol. 63, no. 3, 28 June 1990 (1990-06-28), pages 439-444, XP009043552 ISSN: 0340-6245 cited in the application
- GRUNKEMEIER J M ET AL: "Fibrinogen adsorption to receptor-like biomaterials made by pre-adsorbing peptides to polystyrene substrates" JOURNAL OF MOLECULAR RECOGNITION, vol. 9, no. 3, 1996, pages 247-257, XP002316720 ISSN: 0952-3499 cited in the application

## Description

The present invention as defined by claims 1-27 relates to platelet substitutes (also called synthetic, or artificial, platelets) that are useful for treating patients with deficiencies in their own platelets, such as hereditary or acquired defects of platelet numbers (thrombocytopenia) or function (thrombasthenia).

The body controls bleeding by forming blood clots. In order that a stable, insoluble, blood clot can form, and stop bleeding, a number of different components, of which the most important are thrombin, fibrinogen and platelets, need to be present at the site of a wound. Damaged tissue at the site of the wound releases tissue factor, which activates the coagulation cascade leading to the production of the enzyme thrombin. Thrombin converts fibrinogen, a soluble plasma protein, to an insoluble polymer, which is an essential part of the clot. Also present at the site of the wound are activated platelets. Platelets are the smallest cellular component of blood, and, once activated, platelets also form an essential part of a blood clot. In an initial step, platelets will adhere to the exposed wound surface and become activated. One of the platelet membrane glycoproteins, GPIIb/IIIa, undergoes a shape change, which allows it to bind fibrinogen. Fibrinogen is bipolar, which means it can bind to more than one platelet, and consequently platelets aggregate together. Platelet aggregates form the basic architecture of the clot, formed within a mesh of fibrin.

It can be seen that in the absence of any one of the three components (thrombin, fibrinogen or platelets), the fibrin clot will fail to form properly and bleeding will fail to stop. Hereditary or acquired defects of platelet numbers (thrombocytopenia) can be caused as a result of decreased production of platelets by the bone marrow, for example in malignancy such as leukaemia or as a result of cytotoxic therapy, or as a result of an increased rate of clearance from the circulation, for example in the case of an immune response to platelet antigens. Hereditary or acquired defects of platelet function (thrombasthenia), for example Glanzmann's thrombasthenia (a defect in the GPIIb-IIIa fibrinogen receptor), or Bernard Soulier syndrome (a defect in the GPIb receptor for von Willebrand factor), or storage pool defects such as Grey Platelet Syndrome, Wiscott-Aldrich Syndrome or Hermanski-Pudlack Syndrome, can lead to poor haemostasis or clinically-significant bleeding.

Platelet transfusion is currently the only effective treatment for acute bleeding and the prevention of bleeding in patients with disorders of platelet production and/or function. The 1997 Consensus Conference on Platelet Transfusion highlighted concerns about the ever-increasing demand for platelets. In the final statement of the conference it was concluded that, while there is extensive clinical evidence that platelet transfusions are valuable, the procedure carries risks and costs, raising the ethically crucial issue of balancing these with benefits.

The current standard preparation of a platelet concentrate is a suspension of platelets in autologous plasma prepared by centrifugation of whole blood (buffy coat preparations) or by apheresis. The shelf life of the concentrates is a balance between the competing needs to maintain platelet function and integrity (for which storage is optimal at 22°C), and to minimise bacterial growth (for which storage is optimal at 4°C). This conflict is resolved by storing platelet concentrates at 22°C, but restricting their shelf life to 5 days to minimise bacterial contamination. However, even over this time platelets become steadily activated. The short duration of storage and increasing clinical demand for new therapeutic regimes are resulting increasingly in shortages in supply worldwide.

Despite stringent conditions of preparation and storage, platelet transfusion is still associated with risk of acute bacterial infection. The very low but finite risk of transmitting blood-borne viruses is also well recognised, and more recently there is recognition of the theoretical risk for transmission of vCJD. This risk has been thought to be associated with leucocytes in the concentrates and may therefore be reduced, or eliminated, by leucodepletion. However, it has been observed that platelets also carry normal prion protein, which is released during storage. Although it is yet to be established whether platelets can also carry the variant prion protein, this is of concern.

The presence of leucocytes in platelet preparations poses additional risks. It increases the risk of immunisation to HLA antigens, which can result in multi-transfused patients becoming refractory to platelets. In addition, on storage, leucocytes can release pyrogenic cytokines, adding to the possibility of an adverse reaction. For these various reasons leucocytes are now routinely depleted from platelet concentrates, but this results in a concomitant reduction in platelet yield and increased cost. In addition, leucodepletion does not remove the issue of platelet-derived cytokines (such as TGF-β and RANTES) that have also been associated with allergic reactions to platelet concentrates.

The problems associated with platelet transfusion have stimulated the search for alternatives and, to date there have been broadly three different approaches.

Attempts have been made to stabilise platelets, or fragments of platelets, to prolong their shelf life, and facilitate the application of bacterial and viral inactivation procedures. Treatment of platelet concentrates with Psoralen, a photochemical agent, and ultraviolet light has been shown to inactivate bacterial and viral pathogens in platelet concentrates. An alternative approach has been to lyophilise preparations of platelet membrane fragments, which have been shown to be transiently, and variably, effective in a limited number of patients. These approaches are still, however, limited by the fact that platelets are a highly variable supply of raw material, which is likely to prove incompatible with reproducible manufacture and quality control.

A second approach is the use of an agent which will stimulate endogenous platelet production for example recombinant growth factors such as, thrombopoeitin or Interleukin 11. Although effective in stimulating endogenous platelet production, this approach is also limited because there is a lag between treatment and the recovery of significant platelet numbers in the blood. As a consequence such therapy is inappropriate for the treatment of an acute bleed. In addition, differences in patient response may result in either underproduction or overproduction of platelets, which can put the patient at risk of bleeding or thrombosis respectively.

The third approach is the development of non-platelet-derived haemostatic agents. The advantage of this approach is the potential to design a sterilised, lyophilised product that can be manufactured cost effectively on a large scale, using non-platelet-derived, biocompatible, specified raw materials. The aim is to develop a particulate material that has the ability to interact with residual platelets at a damaged site in a blood vessel, whilst not inducing a thrombotic reaction in the absence of vascular trauma. To do this it is important to design a product that mimics closely the action of native platelets. Thus an understanding of normal platelet function is a prerequisite to the development of the product, since to be effective a platelet substitute must be able to mimic the key processes in the formation of a haemostatic platelet plug. Furthermore a thrombus so formed must be capable of dissolution by normal fibrinolysis.

Platelets normally circulate in a resting state but, following vessel injury, they rapidly adhere to von Willebrand factor (vWF) on the damaged sub-endothelial cell surface, through the GPIbα platelet receptor. This interaction occurs under conditions of high shear, such as is experienced in flow in damaged blood vessels, and its importance is demonstrated by the bleeding diathesis seen in patients with Bernard-Soulier syndrome (who lack the GPIb receptor) or severe von Willebrand's disease (who lack vWF). The process of this adhesion, together with the presence of a range of platelet agonists (collagen in the vessel wall, ADP released from damaged cells, thrombin generated locally by the interaction of exposed tissue factor with plasma clotting factors), causes activation of the platelets. This results in a conformational change in the GPIIb-IIIa receptor complex, allowing it to bind plasma fibrinogen and recruit further platelets into a growing thrombus. Platelets can also expose a negatively charged surface to which the prothrombinase complex can bind and generate thrombin, thus adding to the haemostatic plug by cleaving fibrinogen to form fibrin.

Platelet-platelet aggregation is critically dependent upon the interaction of fibrinogen with GPIIb-IIIa - the "final common pathway" of platelet activation. Coller (1980) Blood 55, 2 demonstrated that inert beads coated with fibrinogen would bind to platelets through the GPIIb-IIIa receptor in the presence or absence of ADP, thus mimicking the action of platelets in causing aggregation.

Several attempts have been made to exploit this concept to develop a platelet substitute. RGD peptides (i.e. peptides comprising the motif Arg-Gly-Asp), designed to interact with the GPIIb-IIIa platelet receptor, have been covalently coupled to erythrocytes. Although the preparation could interact with activated platelets under conditions of low shear, it failed to reduce bleeding in thrombocytopenic primates (Alving et al, 1997, Transfusion, 37, 866-876). In a separate study, fibrinogen cross-linked to erythrocytes with formaldehyde augmented ADP- and thrombin-induced platelet aggregation, and shortened the bleeding time in thrombocytopenic rats (Agam & Livne, 1992, Eu. J. Clin. Invest., 22, 105-112). Whilst offering some promise, both of these approaches have the disadvantage of still relying on cellular material.

WO 98/17319 discloses a product consisting of fibrinogen coated upon the surface of microcapsules of cross-linked human serum albumin. These were shown to interact with platelets under conditions of high shear, and significantly reduced the bleeding in thrombocytopenic rabbits. The fibrinogen-coated microcapsules were shown to interact with the GPIIb-IIIa receptor, because their binding to platelets was inhibited by an RGD-containing peptide. Partial blocking of activity by hirudin indicated that thrombin had cleaved the immobilised fibrinogen. The fibrinogen-coated microcapsules aggregated platelets in the presence of an agonist but were also able to induce platelet aggregation in the absence of agonists (i.e. were thrombogenic). This latter effect was variable and was batch and platelet donor dependent. Thus, although these studies indicated that these microcapsules could augment haemostatic plug formation, their interaction with non-activated platelets is an issue and could lead to adverse thrombotic *events in vivo.* It was clear from these data that further development of the product was required.

Additional analysis of the product of WO 98/17319 is described in Davies et al (Platelets, 2002, 13, 197), in which the WO 98/17319 product is referred to as a "Synthocytes™". The ability of Synthocytes™ to induce platelet aggregation was analysed in inactive and activated platelets, i.e. in the absence or presence of the platelet activation agonist, ADP, respectively. Figure 2(A) of *Davies et al* reports the results of platelet aggregation assays in whole blood (WB), as measured by platelet counting techniques. Figure 2(A) shows that platelet aggregation for inactivate platelets (i.e. in the absence of ADP) is about 20% without Synthocytes™ and about 50% in the presence of Synthocytes™. In other words, Synthocytes™ cause increased aggregation of inactive platelets. Under the specific test conditions, the increase in aggregation of inactive platelets was an approximate 2.5-fold. Figure 2(A) also shows the effects of Synthocytes™ on activated platelets. Platelet aggregation of activated platelets (i.e. in the presence of ADP) is about 40% in the absence of Synthocytes™ and about 70% in the presence of Synthocytes™. This is a less than 2-fold increase in platelet activation. Figure 2(A) of Davies *et al* demonstrates that Synthocytes™ have a platelet aggregating activity in the absence of ADP, i.e. they constitutively aggregate inactive platelets. Moreover, Figure 2(A) shows that Synthocytes™ cause a greater increase in the aggregation of inactive platelets (approximately 2.5-fold) than of activated platelets (less than 2-fold). These data demonstrate that Synthocytes™ do not bind (i.e. aggregate) activated platelets in preference to inactive platelets. Rather, the Synthocytes™ of WO 98/17319 are constitutively active in the aggregation of platelets, irrespective of whether the platelets are active or inactive.

WO 99/25383 describes a pharmaceutical conjugate comprising an insoluble carrier to which first and second active agents are bound, respectively via a first linker to a first functional group on the carrier and via a second linker to a second functional group on the carrier.

WO 99/42146 describes a product comprising thrombin and microparticles having bound fibrinogen, as a combined preparation for simultaneous use in wound therapy or surgical repair.

EP 0618225 describes linear peptides of the formula I: X-A-B-C-Arg-E-G-L-Z. These compounds act as integrin inhibitors and can be used in particular for the prophylaxis and treatment of circulatory disorders and in tumour therapy.

WO 2004/045542 describes a therapeutic bioconjugate composed of a hydrophilic polymer covalently bound to one or more peptides capable of binding specifically to a ligand expressed no a cell surface and thereby forming a biofilm to prevent attachment of cells with the binding partner of the ligand.

The object of the invention as defined by claims 1-27 is thus to provide an improved platelet substitute. In particular, it is an object of the invention to addresses the need in the prior art for a safe non-thrombogenic platelet substitute.

### Description of the Invention

The present invention as defined by claims 1-27 provides an injectable pharmaceutical product comprising an agent, the agent comprising an insoluble carrier to which is bound a peptide, the peptide being capable of binding fibrinogen such that the agent binds via the bound fibrinogen to activated platelets in preference to inactive platelets, and wherein the peptide is not fibrinogen.

In one embodiment, the peptide binds to the region of fibrinogen that is naturally bound either by the platelet membrane glycoproteins GPIIb-IIIa or by fibrin.

In a preferred embodiment, the peptide binds to the region of fibrinogen that is naturally bound by GPIIb-IIIa. The binding of GPIIb-IIIa to fibrinogen is discussed in Bennett, 2001, Annals of NY Acad. Sci., 936, 340-354.

The peptide may bind to one or both of the carboxy- or amino-terminal domains of the α-chain of fibrinogen. More particularly, the peptide may bind to an RGD-containing motif in one or both of said domains. The RGD-containing motif may have the sequence RGDX (SEQ. ID NO:1), where X is any amino acid, such as serine, valine, phenylalanine or alanine, and thus may be RGDF (SEQ. ID NO:2) at amino acids 95-98, or RGDS (SEQ. ID NO:3) at amino acids 572-575.

The peptide may bind to the C-terminal domain of the γ-chain of fibrinogen. More particularly the peptide may bind to a sequence within the final 15, 12, 10 or 4 amino acids of the C-terminal domain of the fibrinogen γ-chain. The final 12 amino acids are usually HHLGGAKQAGDV (SEQ. ID NO:4).

In another preferred embodiment, the peptide binds to the region of fibrinogen that is naturally bound by fibrin. Fibrin binding to fibrinogen is discussed in Mosesson et al, 2001, Ann. N.Y. Acad. Sci., 936, 11-30, the contents of which are incorporated herein by reference. The peptide may bind the D-domain of the γ-chain, such as between residues 337-379. The peptide may bind to the β-chain segment of the D-domain, such as the C-terminal region.

The peptide may bind fibrinogen with a dissociation constant (*K_{d}*) of around 10, 20, 30, 40, 50, 60, 70, 80, 90, 100, 110, 120, 130, 140, 150, 200, 250, 300, 350, 400 or more nM. A *K_{d}* of around 100 nM is preferred.

An agent of the invention as defined by claims 1-27 binds fibrinogen such that, when bound, the fibrinogen binds activated platelets in preference to inactive platelets. Activated platelets are platelets in which changes resulting from stimulation by an agonist causes a change in the conformation of GPIIb-IIIa which then allows fibrinogen to bind and thus allows the platelets to aggregate, and in some cases release the contents of their intracellular granules, for example 5HT or to express granule membrane proteins on their surface, for example α-granule P selectin. The precise nature of the response varies between agonists and according to the dose of the agonist. Examples of agonists are thrombin, ADP and collagen. Platelets that are not activated have the potential to undergo such changes but have not yet been stimulated to do so by an agonist.

To test whether a peptide is capable of binding fibrinogen such that the fibrinogen has a binding preference for activated platelets, the test peptide is bound to a carrier according to the present invention as defined by claims 1-27 and fibrinogen is allowed to bind the peptide, as described below, thereby to generate a test agent. The test agent is added to platelets in suspension, for example in whole blood, platelet rich plasma or a suitable buffer solution in the presence or absence of an agonist of platelet activation, such as ADP (i.e. the test agent is added to activated or inactive platelets, respectively), and gently mixed, as described in Davies et al, 2002, Platelets, 13, 197. The platelet suspension/test agent mixture is then analysed to determine whether the platelets are aggregated, for example by using the platelet counting technique described in the following examples or in Davies *et al.* As a control, the level of platelet aggregation is determined in the presence or absence of ADP, without adding the test agent, such as described in the following examples or in Davies *et al.* The level of platelet aggregation correlates with the ability of the bound fibrinogen to bind a platelet. An agent according to the present invention as defined by claims 1-27, having a peptide that is capable of binding fibrinogen such that the fibrinogen has a binding preference for activated platelets, will show a bigger increase in aggregation between control and agent in the presence of ADP than in the absence of ADP. The skilled person will appreciate that the same test can be performed using agonists of platelet activation other than ADP.

Typically, an agent according to the present invention as defined by claims 1-27, having a peptide that is capable of binding fibrinogen such that the fibrinogen has a binding preference for activated platelets, will cause less than a 150% increase in aggregation of inactive platelets, such as less than 140%, 130%, 120%, 110%, 100%, 90%, 80%, 70%, 60%, 50%, 40%, 30%, 20%, 10%, 5%, 4%, 3%, 2%, 1% or substantially no increase in the aggregation of inactivated platelets compared to the inactive control level. Lower numbers are preferred. In this context, the basal level of aggregation of inactive platelets is taken to be 100%, and thus a 100% increase as defined above is a doubling (i.e. two-fold increase) in the level of aggregation and a 150% increase is a 2.5-fold increase in the level of aggregation.

An agent according to the present invention as defined by claims 1-27, having a peptide that is capable of binding fibrinogen such that the fibrinogen has a binding preference for activated platelets, may cause at least a 5%, 10%, 20%, 30%, 40%, 50%, 60%, 70%, 80%, 90%, 100% (i.e. 2-fold) increase, or more, in the aggregation of activated platelets compared to the activated control. Higher numbers are preferred. The level of increase in aggregation of activated platelets caused by an agent of the invention may be up to 50%, 60%, 70%, 80%, 90%, 100%, 150%, 200%, 250%, 300%, 350%, 400%, 450%, 500% or more.

In one embodiment, it is preferred that the agent of the invention as defined by claims 1-27 is able to cause a greater fold-increase in the aggregation of activated platelets, compared to the activated control (i.e. activated platelets in the absence of the agent of the invention), than the inactive platelets, compared to the inactive control (i.e. inactive platelets in the absence of the agent of the invention).

A product is an injectable pharmaceutical product if it is sterile, substantially pyrogen-free and has no medically unacceptable effects. For example, the product should not produce a medically unacceptable immunological reaction when injected into a human subject. Medically unacceptable effects can be determined by the skilled person in the field of medicine.

Importantly, in the case of the present invention as defined by claims 1-27, the fibrinogen-binding peptide of the agent should be capable of binding fibrinogen such that, if the agent has been loaded with fibrinogen via the fibrinogen-binding peptide and administered to a patient intravenously, the fibrinogen bound to the agent via the peptide should not be active in the formation of medically unacceptable levels of non-specific fibrin clots. By "non-specific", in this context, we include fibrin clot formation that occurs in the absence of active platelets at the site of a wound.

As discussed above, WO 98/17319 discloses fibrinogen-coated microcapsules, which induce platelet aggregation of inactive platelets (i.e. they are thrombogenic products). This is medically unacceptable. Without being bound by theory, we believe that the product of the present invention as defined by claims 1-27 addresses the disadvantages faced by the prior art by binding fibrinogen in a conformation that does not result in medically unacceptable constitutive fibrinogen action. Accordingly, the fibrinogen-binding peptide bound directly to the carrier as used in the product of the invention is not fibrinogen, as defined in WO 98/17319.

*In vivo,* fibrinogen typically binds to platelets that are activated by the presence of an agonist, such as ADP, thrombin, or collagen.

In one embodiment, if administered to a patient intravenously, the product will preferentially become involved in formation of a blood clot at the site of a wound where platelets are already activated. In this context, the phrase "preferentially becomes involved" means that, although low levels of binding of the product to inactive platelets may be acceptable, that level will not cause medically unacceptable levels of fibrin clot formation.

The fibrinogen-binding peptide as used in the product may comprise a sequence obtained from the platelet membrane glycoproteins GPIIb or GPIIIa (Bennett, 2001, Annals of NY Acad. Sci., 936, 340-354).

In particular, the fibrinogen-binding peptide may be obtained from fibrinogen-binding regions of GPIIb or GPIIIa. Preferred fibrinogen-binding regions include regions, which bind the α-chain amino, and/or carboxy-terminal domains of fibrinogen and regions that bind the γ-chain C-terminal domain of fibrinogen, as discussed above.

Thus the fibrinogen-binding peptide may comprise the sequence of AVTDVNGDRHDLLVGAPLYM (SEQ. ID NO:5), which represents the sequence of amino acids 294-314 of GPIIb, or a fibrinogen-binding fragment thereof. Such fragments include the sequence TDVNGDGRHDL (SEQ. ID NO:6) (296-306), the sequence GDGRHDLLVGAPLL (SEQ. ID NO:7) (300-312) and the terminal tetrapeptide GAPL (SEQ. ID NO:8). These sequences are thought to be involved in the binding of fibrinogen and, in particular, the γ-chain of fibrinogen (Bennett, 2001, *op. cit.;* D'Souza et al, 1991, Nature, 350, 66-68; Taylor & Gartner, 1992, J. Biol. Chem., 267, 11729-33). The similar effects of fragments 296-306 and 300-312 suggest that fragment 300-306 may also provide fibrinogen-binding activity.

Grunkemeier et al (1996, J. Molecular Recognition, 9, 247-257) reported that purified TDVNGDGRHDL (SEQ. ID NO:6) (designated "B12") peptide caused inhibition of platelet aggregation. Grunkemeier *et al* used this information to propose non-platelet-adhesive materials coated in B12 peptide, and hypothesised that B 12 would bind fibrinogen specifically in the region that binds to the GPIIb/IIIa platelet receptor, thus blocking platelet aggregation. Therefore, the understanding in Grunkemeier *et al* is that, when immobilised, the B12 peptide can be used to block fibrinogen binding to platelets, and thus inhibit platelet aggregation. In light of this teaching, it was not apparent that the B12 peptide would be suitable for use in a platelet substitute for aiding platelet aggregation and blood clot formation.

The fibrinogen-binding peptide may comprise one or more of the peptides APLHK (SEQ. ID NO:9), EHIPA (SEQ. ID NO:10) and GAPL (SEQ. ID NO:8) which were shown in Gartner, 1991, Biochem. Biophys. Res. Commun., 180(3), 1446-52 to be hydropathically equivalent peptide mimics of the fibrinogen binding domain of GPIIb-IIIa.

The fibrinogen-binding peptide may comprise the sequence of residues 95-223 of GPIIIa or a fibrinogen-binding fragment thereof. For example, residues 211-222, comprising the sequence SVSRNRDAPEGG (SEQ. ID NO:11) is thought to be an important fibrinogen-binding domain in GPIIIa (Charo et al, 1991, J. Biol. Chem., 266, 1415-1421).

Other suitable regions of GPIIIa include residues 109-171 and 164-202.

The skilled person will appreciate that fragments or variants of any of these sequences may also be used, so long as they provide fibrinogen-binding activity according to the present invention as defined by claims 1-27.

A particularly preferred fibrinogen-binding peptide comprises a sequence obtained from the platelet membrane glycoprotein GPIIb, namely TDVNGDGRHDL (SEQ. ID NO:6), or a variant of such a sequence.

Variants of TDVNGDGRHDL (SEQ. ID NO:6) include -
T(D,E)VNG(D,E)GRH(D,E)L (SEQ**.** ID NO: 12.)
TD(V, L)NGDGRHDL (SEQ. ID NO:13)
TDV(N,Q)GDGRHDL (SEQ. ID NO: 14)
TDVNGDG(R,K)HDL (SEQ. ID NO: 15)

Such variants will have substantially the same fibrinogen binding activity as TDVNGDGRHDL (SEQ. ID NO:6), in that they will have substantially the same affinity for fibrinogen and, when bound, fibrinogen will have substantially the same conformation and activity as when bound to TDVNGDGRHDL (SEQ. ID NO:6). By "substantially the same fibrinogen-binding activity" we include variants that bind fibrinogen with an affinity up to 1, 2, 3, 4, 5, 10, 50, 100 or more orders of magnitude different (either higher or lower) to TDVNGDGRHDL (SEQ. ID NO: 6). Lower numbers are preferred.

Kuyas et al, 1990, Thrombosis and Haemostasis, 63(3), 439, describes the use of the synthetic peptide GPRPK (SEQ. ID NO:6), immobilised via the C-terminal lysine to fractogel, to isolate fibrinogen from human plasma. Kuyas *et al* explains that human fibrinogen has a strong affinity for fibrin, and reports that the authors utilised a peptide comprising the N-terminal sequence of the α- chain of fibrin exposed by the action of thrombin, GPRP (SEQ. ID NO:17), which had been shown to bind fibrinogen (Laudano & Doolittle, 1980, Biochemistry, 19, 1013; Laudano et al, 1983, Ann. N.Y. Acad Sci., 408, 315). Kuyas *et al* concludes that the 'core' sequence GPR is required for fibrinogen binding.

Thus, the fibrinogen-binding peptide as used in the product may comprise the sequence of a fibrinogen-binding region of fibrin such as the N-terminal region of the α-chain or the C-terminal region of the β-chain. Accordingly the peptide may have the sequence Gly-(Pro/His/Val)-Arg-Xaa (SEQ. ID NO:18) at the amino terminus, wherein Xaa is any amino acid. In this context, by "at the amino terminus" we mean that the Gly residue in the above tetrapeptide sequence should represent the first amino acid of the peptide when read from the N-terminus to the C-terminus. By "Pro/His/Val" we mean that either proline, histidine or valine is included at that position. In one embodiment, proline and histidine are preferred, and proline is most preferred.

Kuyas *et al* fails to disclose an injectable pharmaceutical product according to the present invention as defined by claims 1-27 because the peptide is bound to Fractogel. Fractogel is composed of polymethacrylate and has a minimum particle size of 20mm and would therefore not be pharmaceutically acceptable.

The peptide may comprise the sequence of Gly-Pro-Arg-Pro (SEQ. ID NO:17) at the amino terminus.

Alternatively, the peptide may comprise the sequence of Gly-Pro-Arg-Sar (SEQ. ID NO:19) (Sar is short for sarcosine, which is methyl glycine), Gly-Pro-Arg-Gly (SEQ. ID NO:19) or Gly-Pro-Arg-Val (SEQ. ID NO:19) at the amino terminus.

The peptide may comprise, in addition to a fibrinogen-binding sequence, an amino acid or sequence designed to aid attachment of the peptide to the carrier. For example, the peptide may include a terminal cysteine for linking to a thiol reactive group on the carrier (see below).

Typically the peptide has from 4 to 200 amino acids. Preferably, the peptide is no more than 150, 100, 90, 80, 70, 60, 50, 40, 30 or 20 amino acids in length. Preferably, the peptide is at least 4, 5, 6, 7, 8, 9, 10, 11 or more amino acids in length, although the minimum length should be at least long enough to include the fibrinogen-binding sequence in full.

The peptide may also comprise a spacer sequence. This can provide for spatial distances between the fibrinogen-binding sequence and the linkage to the carrier. This may aid in preserving the fibrinogen-binding activity of the peptide. For example, a spacer sequence of 1, 2, 3, 4, 5, 6, 7, 8, 9, 10, 11, 12, 13, 14, 15, 16, 17, 18, 19, 20 or more amino acids may be suitable. The sequence of the spacer may comprise a mix of amino acids or be a repeat of a single amino acid. For example, a poly(glycine) sequence may be suitable for use as a spacer.

The carrier should be insoluble, inert and biocompatible. The carrier should exhibit an insignificant effect on blood coagulation tested by adding the carrier to plasma and demonstrating no effect on the activated partial thromboplastin clotting time (APPT) using for example micronized kaolin (supplied by Helena Laboratories Ltd.) to activate recalcified plasma or the prothrombin clotting time (PT) using for example Manchester thromboplastin reagent (supplied by Helena Laboratories). Similarly, the carrier should exhibit no effect on platelets when tested by the method of Davies et al, 2002, Platelets, 13, 197 as described above. The phrase "no effect" as used above includes the meaning that the carrier has no medically unacceptable effect, as described above.

The carrier should have a size suitable to ensure transmission of the agent through the lung capillary bed. The ability of an agent to be transmitted through the lung capillary bed can be determined using the method of Perkins et al, 1997, The British Journal of Radiology, 70, 603. Alternatively the ability of an agent to be transmitted through the lung capillary bed can be determined by injecting the agent into a host, for example an anaesthetised dog or cynamolgous monkey, and studying cardiovascular and respiratory safety, including an analysis of parameters such as blood pressure, pulse oximetry, respiratory and heart rate, and blood gas analysis. An agent that is able to be transmitted through the lung capillary bed, when injected into the host, will have substantially no effect on these parameters.

In this embodiment, the carrier may have a maximum dimension such that a minority, such as less than about 2% of the population by number, are in excess of 6 µm as a maximum dimension, as measured by particle counter, such as a Coulter Multizer II. A size of from 2 to 4 µm as a maximum dimension may be suitable, which is comparable to the size of human platelets.

In one embodiment, the carrier may be a microparticle. The term "microparticle" includes solid, hollow and porous microparticles. The microparticles may be spherical (i.e. be "microspheres"), by which we include all substantially spherical shapes.

The microparticle may be formed of any suitable substance. It may be formed of cross-linked protein. A typical protein for these purposes is albumin, which may be serum-derived or recombinant and may be human or non-human in sequence. A protein microparticle may be formed by spray-drying protein. For example, microparticles suitable for use as a carrier by the present invention may be formed by spray drying human serum albumin, using well known spray-drying technology, such as in WO 92/18164. Accordingly, the carrier may be an albumin microparticle.

Alternatives to the use of microparticles as carriers include liposomes, synthetic polymer particles (such as polylactic acid, polyglycolic acid and poly(lactic/glycolic) acid), cell membrane fragments and the like.

The peptide can be bound to the carrier by any suitable means. The bond between the peptide and the carrier can be covalent or non-covalent. Typically the bond is covalent. A suitable covalent bond can be formed when the peptide comprises a cysteine and the carrier comprises a thiol reactive group. This allows the peptide to be bound to the carrier by linking the -SH group of the cysteine to the thiol reactive group on the carrier. As discussed above, a terminal cysteine residue may be incorporated in the fibrinogen-binding peptide to crosslink the peptide with thiol reactive groups on the carrier. For example the free thiol on an albumin carrier can be used, a leaving group (for example 5,5'-dithio-bis(2-nitrobenzoic acid) (DTNB) or Ellman's reagent) can be substituted into the free suphydryl group on the albumin carrier, and the cysteine in the peptide substituted for the leaving group. Suitable thiol reactive groups also include maleimide as disclosed in Green et al, 1982, Cell, 28, 477, although the skilled person will appreciate that any suitable method can be used.

For example thiol reactive groups can also be made available on the carrier using, for example, maleimidobenzoly-N-hydroxsuccininmide ester (MBS) or succinimidyl 4-(N-maleimidomethyl)cyclohexane-1-carboaylate) (SMCC) crosslinkers to convert lysine residues in the carrier to thiol reactive maleimide groups.

An alternative method of linking the peptides to the carrier is to use a two step carbodiimide method (Grabarek, 1990, Analytical Biochemistry, 185) in which the peptide is incubated with 1-ethyl-3-(3-dimethylaminopropyl) carbodiimide hydrochloride (EDC) and N- hydroxysulphosuccinimide (sulpho-NHS), which results in the formation of an active ester. The peptide ester can be isolated and then mixed with the carrier and the ester allowed to react with amines on the carrier.

Any number of fibrinogen-binding peptides may be bound to the carrier. A platelet typically has 50,000-100,000 GPIIb-IIIa surface proteins. A similar number of fibrinogen-binding peptides on the carrier may be appropriate. For example, the carrier may have at least 1,000, 5,000, 10,000, 20,000, 30,000, 40,000, 50,000, 60,000, 70,000, 80,000, 90,000, 100,000 or more fibrinogen binding peptides bound thereto, such as up to 50, 80, 90, 100, 110, 120, 130, 140, 150, 200, 400 or more thousand fibrinogen binding peptides. In one embodiment, the number of fibrinogen-binding peptides may be around 5,000, 10,000, 20,000, 30,000, 40,000, 50,000, 60,000, 70,000, 80,000, 90,000, 100,000 or more.

Fibrinogen, or a variant or fragment thereof, may be bound to the thus formed product, to provide an immobilised form of fibrinogen for administration to an individual.

This may be achieved by a method comprising the steps of providing a product as described above and mixing it with fibrinogen, or a variant of fragment thereof. In one embodiment, the fibrinogen (or variant or fragment) binds to the peptide as a result of the affinity of the peptide for the fibrinogen (or variant or fragment). Thus, the fibrinogen (or variant or fragment) may be bound to the peptide by non-covalent bonds. The non-covalent bonds can subsequently be stabilised by the formation of an additional covalent bond between the fibrinogen (or variant or fragment) and the peptide, or between the fibrinogen (or variant or fragment) and the carrier. Alternatively, the sole means of attachment of the fibrinogen (or variant or fragment) can be through a covalent bond to the peptide.

One suitable method for non-covalently attaching fibrinogen (or variant or fragment) includes incubating a product as defined above with blood, or plasma or a concentrate of plasma-derived or recombinant fibrinogen, which is suitable for intravenous use at between 20°C and 37°C for an appropriate length of time. We have found that incubation for up to 3 hours at 20°C is satisfactory. Further methods of non-covalently attaching fibrinogen are discussed in the Examples below.

The amount of fibrinogen bound to the product can be varied to obtain desired product characteristics. Typically, the product is incubated with a fibrinogen solution at a fibrinogen concentration, and under conditions, suitable to achieve a product in which at least 10%, preferably at least 20%, 30%, 40%, 50%, 60%, 70%, 80%, 90%, 95%, 99% or more of the particles in the product have non-covalently bound fibrinogen. In one embodiment, the fibrinogen incubation conditions chosen result in products having a median fluorescence intensiy (MFI) of at least 1, 2, 3, 4, 5, 6, 7, 8, 9, 10, 11, 12, 13, 14, 15, 16, 17, 18, 19, 20, 21, 22, 23, 24, 25, 26, 27, 28, 29, 30 or more, as determined by a method as disclosed in the following examples. More preferably, the MFI is less than 31, 30, 29, 28, 27, 26, 25, 24, 23, 22, 21, 20, 18, 19, 17, 16, 15, 14, 13, 12, 11, 10, 9, 8, 7, 6, or 5.

To achieve covalent attachment, fibrinogen can be crosslinked to the peptide, for example, using a zero length heterobifunctional crosslinker, such as EDC plus Sulpho-NHS as discussed above.

Subsequent steps in the production of an injectable pharmaceutical composition may include -
(a) removing unbound fibrinogen;
(b) formulating the product with a pharmaceutically acceptable carrier or diluent;
(c) diluting the product to provide a pharmaceutically acceptable unit dose; and
(d) sterilising the product.

Specifically steps in production may be carried out as follows -
(a) Unbound fibrinogen may be removed by centrifugation at 2000 × *g* for 15 minutes at 20°C, and the product subsequently washed by resuspending in an isotonic buffer (for example, 50mM sodium phosphate buffer containing 0.15M sodium chloride at pH 7.0-7.4, or 0.02M Tris containing 0.15M sodium chloride pH 7.0-7.4). Alternatively the product may be washed with one of the above buffers using tangential ultrafiltration, when the product will retained by the membrane.
(b) The product may be formulated by resuspending in an isotonic buffer at a physiological pH (for example, using one of the buffers described above). A buffer containing, for example, mannitol or glucose could also be used. In one embodiment the product can be lyophilised, to produce a freeze-dried dosage form, which can be reconstituted immediately prior to use. A suitable dosage form might be between 0.5 gram and 5 gram of product.

It will be appreciated that steps (b) and (c) can be the same or different.

It will also be appreciate that step (d) may not be required. The product is typically produced aseptically and, in a preferred embodiment, is additionally subjected to a terminal heat treatment. An example of a suitable terminal heat treatment of a liquid suspension is heating at a suitable temperature, for example 60°C, for 10 hours. Alternatively, the product can be first lyophilised and then heated to, for example, 80°C for 72 hours. Such procedures are commonly used to destroy viruses in blood proteins and would be expected to destroy bacteria. Alternatively, the heat treatment step could be replaced by gamma irradiation, for example by exposure to 25-35Kgy using a cobalt⁶⁰ source.

The form of attachment can be varied so long as the bound fibrinogen (or variant or fragment) binds to activated platelets in preference to inactive platelets, and preferably, following intravenous administration, will only become involved in the formation of a blood clot at the site of a wound where platelets are already activated.

Accordingly, the product may additionally comprise fibrinogen, or a variant or fragment thereof, having an inducible platelet-aggregating activity, bound to the said peptide.

By "inducible platelet-aggregating activity", we mean that the fibrinogen binds to activated platelets in preference to inactive platelets. Preferably, if administered to a patient intravenously, the fibrinogen portion of the product will preferentially become involved in formation of a blood clot at the site of a wound where platelets are already activated. Methods for determining whether the platelet-aggregating activity of fibrinogen is "inducible" are discussed above.

The source of the fibrinogen can be, for example, a purified protein derived from plasma or blood or from a recombinant source. The fibrinogen may be human or non-human in sequence.

Any variant or fragment of fibrinogen may be used, provided that it has a useful level of inducible platelet-aggregating activity. In this context, a useful level of inducible platelet-aggregating activity means that the variant or fragment can be used with the product of the invention to cause aggregation of activated platelets in preference to inactive platelets, as described above. Preferably, any such variant or fragment includes residues 398-411 of the gamma chain of fibrinogen. In a preferred embodiment, the variant or fragment may include, or even consist of, HHLGGAKQADV (SEQ. ID NO:20)

Accordingly, the present invention also provides an injectable pharmaceutical product having an inducible platelet-aggregating activity comprising an insoluble carrier to which fibrinogen, or a variant or fragment thereof, is bound in a configuration such that the fibrinogen (or variant or fragment) binds to activated platelets in preference to inactive platelets.

Typically the product, when introduced intravenously, will only become involved in formation of a blood clot at the site of a wound where platelets are already activated. The fibrinogen, or a variant or fragment thereof, is typically bound indirectly to the carrier through a fibrinogen-binding peptide as defined above.

In an alternative embodiment, a product as defined above may be administered without fibrinogen. In this case, the product is able to bind fibrinogen endogenous to the individual to whom the product is administered.

Accordingly, the present invention as defined by claims 1-27 also provides a method of promoting haemostasis, i.e. improving the ability of an individual to produce fibrin clots, comprising administering a pharmaceutically effective dosage of a product as defined above. The product can thus be used to promote an individual's ability to form fibrin clots at wound sites, whilst avoiding medically unacceptable levels of non-specific formation of fibrin clots away from wound sites. Typically the method is a method of treating a patient with thrombocytopenia

Thrombocytopenia may result from conditions that cause increased platelet destruction. These include Immune thrombocytopenic purpura, disseminated intravascular coagulation, heparin-induced thrombocytopenia, other drug-induced thrombocytopenias, systemic lupus erythematosus, HIV-1-related thrombocytopenia, thrombotic thrombocytopenia purpura/haemolytic-uremic syndrome, common variable immunodeficiency, post-transfusional purpura, and type 2B von Willebrands disease.

Thrombocytopenia may result from conditions that cause decreased platelet production. These include thrombocytopenia with absent radii (TAR) syndrome, amegakaryocytic thrombocytopenia, giant platelet syndromes (such as Bernard-Soulier syndrome, May-Hegglin anomaly, Fechtner syndrome, Sebastian syndrome, Epstein syndrome, Montreal platelet syndrome), and Wiskott-Aldrich syndrome.

Thrombocytopenia may result from conditions that cause sequestration (for example, hypersplenism or Nasabach-Merritt syndrome) or increased platelet destruction and hemodilution (such as extracorporeal perfusion).

The method of the invention as defined by claims 1-27 may also be used to treat a patient with any one of the above conditions.

However, the method may also be used to treat a patient with thrombasthenia (i.e. inherited or acquired). Acquired platelet function defects can result from uremia, myeloproliferative disorders (such as essential thrombocythemia, polycythemia vera, chronic myeloid leukaemia, and agnogenicmyeloid metaplasia), acute leukaemias and myelodysplatic syndromes, dysproteinemias, extracorporeal perfusion, acquired von Willebrands disease, acquired storage pool deficiency, antiplatelet antibodies, liver disease, drugs and other agents. Inherited platelet function defects can result from platelet adhesion conditions (such as Bernard-Soulier syndrome and von Willebrand disease), agonist receptor conditions (such as integrin α₂β₁ (collagen receptor) deficiency, P2Y₁₂ (ADP receptor) deficiency or thromboxane A₂ receptor deficiency), signalling pathway conditions (such as G_{αq} deficiency, phospholipase C-β₂ deficiency, cyclooxygenase deficiency, thromboxane synthetase deficiency, lipoxygenase deficiency or defects in calcium mobilisation), secretion conditions (such as storage pool disease, Hermansky-Pudlak syndrome, Chediak-Higashi syndrome, Gray platelet syndrome, Quebec syndrome and Wiskott-Aldrich syndrome), aggregation conditions (such as Glanzmann thrombasthenia or congenital afibringenemia) and platelet-coagulant protein interaction conditions (such as Scott syndrome).

The method may also be used to treat a patient who has sustained mechanical damage to his/her platelets, such as occurs during extra corporeal circulation in coronary bypass surgery and/or haemodialysis.

Michelson, 2002, Platelets, Chapter 36: The Clinical Approach to Disorders of Platelet Number and Function, Elsevier Science (USA), 541-545, the contents of which are incorporated herein by reference, provides a review of various disorders of platelet number and function.

The present invention as defined by claims 1-27 thus provides products as defined above for use in medicine. The present invention also provides products as defined above in the manufacture of a medicament for promoting haemostasis.

For example, the present invention as defined by claims 1-27 also provides products as defined above in the manufacture of a medicament for the treatment of a patient with thrombocytopenic condition, such as a condition described above.

Thrombocytopenia is diagnosed by counting blood cells. The normal platelet count is 150-400x10⁹/l. Below this range primary haemostasis is impaired and bleeding time prolonged. However spontaneous life threatening bleeding will usually only occur when the platelet count drops under 10X 10⁹/l.

Accordingly, a method or use as defined above can be applied when wherein the patient has a platelet count below 400x10⁹/l, preferably below 150x10⁹/l, and more preferably below 10x 10⁹/l.

The most common cause of thrombocyopenia is a failure in platelet production from the bone marrow, such as in blood cancers or following cytotoxic chemotherapy or radiotherapy.

Accordingly, a method or use as defined above can be applied when the patient has a failure in platelet production from the bone marrow, such as is caused by a blood cancer, or cytotoxic chemotherapy or radiotherapy.

A method or use as defined above can be applied when the patient has an inherited or drug-induced disorders in platelet function, such as described above.

A method or use as defined above can be applied when the patient's platelets have been mechanically damaged, such as occurs during extra corporeal circulation in coronary bypass surgery or haemodialysis.

By "treat" as used above, we include the use of the above products in prophylaxis. Thus, for example, a product of the invention could be administered to a patient in advance of cytotoxic chemotherapy or radiotherapy, drug-induced disorders in platelet function, extra corporeal circulation in coronary bypass surgery or haemodialysis.

The present invention as defined by claims 1-27 is further described in the following, non-limiting, examples.

### Schedule of SEQ ID Nos.

1. RGDX where X is any amino acid
2. RGDF
3. RGDS
4. HHLGGAKQAGDV
5. AVTDVNGDRHDLLVGAPLYM
6. TDVNGDGRHDL
7. GDGRHDLLVGAPL
8. GAPL
9. APLHK
10. EHIPA
11. SVSRNRDAPEGG
12. T(D,E)VNG(D,E)GRH(D,E)L
13. TD(V,L)NGDGRHDL
14. TDV(N,Q)GDGRHDL
15. TDVNGDG(R,K)HDL
16. GPRPK
17. GPRP
18. G(P,H,V)RX where X is any amino acid
19. GPR(X,G,V) where X is methylglycine (sarcosine)
20. HHLGGAKQADV
21. GPRPC
22. GPRPGGGC
23. GPRPGGGGGGC
24. G(P,H)RX where X is any amino acid
25. G(P,H)R(P,X,G,V) where X is methylglycine (sarcosine)

### Example 1: Production of fibrinogen-linked particles (FLPs) and measurement of bound fibrinogen

### 1. General method for producing Fibrinogen linked microspheres, using the peptide GPRP with a spacer consisting of 0, 3 or 6 glycine residues.

(i) A 10mg albumin/ml suspension of washed human albumin microspheres was prepared in an isotonic buffer at pH 7.4 (e.g. phosphate buffered saline). The albumin microspheres used were of a diameter suitable for intravenous use and therefore less than 6 µm. Suitable albumin microspheres are known in the art. For example, WO 03/015756 discloses albumin microparticles that have a diameter of 2-3 µm, pH 7.4.
(ii) 30µl of 10mM 5,5'-Dithio-bis(2-Nitrobenzoic acid) (DTNB) was added to 10mg of microspheres and mixed at room temperature for 2 hours. The microspheres were then separated from the supernatant and washed at least twice in buffer (we used phosphate buffered saline), using centrifugation at 3000 x g for at least 5 minutes.
(iii) The pellet was resuspended in 1 ml of a suitable buffer such as phosphate buffered saline. The peptide (either GPRPC (SEQ. ID NO:21), GPRPGGGC (SEQ. ID NO:22) or GPRPGGGGGGC (SEQ. ID NO:23)), supplied by Merck Biosciences, was dissolved in phosphate buffered saline and added to the microspheres at a final concentration of 0.23mM. This was mixed at room temperature for 24 hours. An appropriate negative control was provided by treating the preparation with 0.23mM L-cysteine (cysteine control). A wash was performed by centrifuging at 3000 x g for 5 minutes and resuspending in phosphate buffered saline. The washing step was repeated twice, followed by resuspension finally in a volume of 1 ml.
(iv) A vial of freeze-dried fibrinogen (for example the material supplied by the Scottish National Blood Transfusion Service) was reconstituted and 3 mg of fibrinogen was added to 1 ml of albumin microspheres obtained from step (iii) and mixed for 1 hour at room temperature. The mix was then centrifuged at 3000 x g for 5 minutes and the supernatant removed. The microspheres were washed three times with phosphate buffered saline as previously described. The final pellet was resuspended at in 1 ml of phosphate buffered saline

### 2. Fibrinogen binding assay

A method to demonstrate fibrinogen binding to the peptide-linked microspheres is described below in which an anti-human fibrinogen FITC-labelled antibody is used to detect fibrinogen bound to the microspheres.
(i) 5µl of a suspension of artificial platelets obtained from step (iv) of method 1 above was added to 50µl of HEPES buffered saline containing 1µl of FITC-labelled rabbit antibody to human fibrinogen (DakoCytomation; F0111). The mixture was incubated at 20°C for 20 minutes. Blank microspheres (without DTNB treatment or peptide) or a cysteine control sample were used as a negative control for these assays.
(ii) After 20 minutes the reaction was stopped by the addition of 0.5ml saline. After a further 10 minutes, the sample was diluted 1/10 in saline and analysed in a Flow Cytometer (e.g. Coulter XL-MCL Flow Cytometer).
(iii) Data were recorded as the percentage of microspheres positive for fibrinogen and the median fluorescence intensity of the particles. The latter is a measure of the amount of fibrinogen bound to the microspheres and is in arbitrary units (log scale).

The products made by method 1 above were tested using method 2. Specifically the products tested had, as the bound peptide, either "peptide Glyⁿ=0" (i.e. GPRPC (SEQ. ID NO:21), "peptide Glyⁿ=3" (i.e. GPRPGGGC (SEQ. ID NO:22)) or "peptide Glyⁿ=6" (i.e. GPRPGGGGGGC (SEQ. ID NO:23)). A cysteine control sample was also included. The results are reported in Table 1 below.

**Table 1: Fibrinogen binding to peptide-linked microspheres**

| **Flow cytometric measure** | **Peptide Glyⁿ = 0** | **Peptide Glyⁿ = 3** | **Peptide Glyⁿ = 6** | **Cysteine control** |
|---|---|---|---|---|
| **Percent positive** | 6 | 94 | 95 | 32 |
| **Median fluorescence intensity** | 2.4 | 28 | 26 | 0.9 |

The results show that the ability of a peptide comprising a GPRP N-terminal sequence to bind fibrinogen can be modified by inclusion of a spacer between the peptide and the microsphere. Without the spacer only 6% of the microspheres carry fibrinogen but with a spacer consisting of 3 or 6 glycine residues, greater than 90% of the microspheres bind fibrinogen.

### Example 2: Further analysis of fibrinogen-linked particles

The following products were produced -
Product 1: Artificial Platelets were produced, using the peptide GPRPGGGGGGC (SEQ. ID NO:23) (i.e. Glyⁿ = 6) with bound fibrinogen, using steps (i) to (iv) of method 1 of Example 1, as discussed above, except that step (iv) used 0.1 mg/ml of fibrinogen, rather than 3 mg/ml.
Product 2: Artificial Platelets were produced, using the peptide GPRPGGGGGGC (SEQ. ID NO:23) (i.e. Glyⁿ = 6) *without fibrinogen,* using steps (i) to (iii) of method 1 as discussed above.
A reference batch: A reference batch of microspheres was prepared using the method described in WO 98/17319. Specifically:
   (i) 1gm of microspheres were washed and suspended in 50ml 0.01M sodium phosphate buffer, pH 6.0.
   (ii) A vial of freeze-dried fibrinogen (for example the material supplied by the Scottish National Blood Transfusion Service) was reconstituted.
   (iii) Fibrinogen was diluted to 10mg/ml using 0.01M sodium phosphate buffer at pH 6.0.
   (iv) 15ml of dilute fibrinogen solution was added per gram of microspheres, and mixed for 4 hours at room temperature (e.g. 20°C).
   (v) The mixture was then centrifuged 2000 x g for 15 minutes at 20°C to pellet the microspheres. The supernatant was removed and the pellet washed in 0.01M sodium phosphate pH 6.0.
   (vi) The mixture was then centrifuged and resuspended in 0.01M sodium phosphate buffer, at least twice until the supernatant was low in protein as determined by absorbance at E280.
   (vii) The microspheres were then finally washed and resuspended at 10mg/ml in phosphate buffered saline pH 7.4.
   (viii) Fibrinogen coated microspheres were then filled into 500ul aliquots and stored at minus 70°C.

In summary, product 1 is a FLP (fibrinogen-linked particle) of the invention having fibrinogen bound via the peptide GPRPGGGGGGC (SEQ. ID NO:23), the peptide being bound to the microsphere. Product 2 is identical to product 1 except that it has no fibrinogen bound to the peptide GPRPGGGGGGC (SEQ. ID NO:23). The reference product has fibrinogen bound directly to the surface of the microsphere, in the manner known from the prior art, such as in WO 98/17319 and Davies *et al, supra.*

These products were tested for fibrinogen binding using method 2 of Example 1, above. The results are reported in table 2 below.

**Table 2: Fibrinogen blinding to Product 1. Product 2 and Reference Batch of microspheres**

| **Flow cytometric measure** | **Product 1** | **Product 2** | **Reference batch** |
|---|---|---|---|
| **Percent positive** | 75 | 13.1 | 94 |
| **Median fluorescence intensity** | 4.6 | 1.6 | 22 |

These results demonstrate that the relative fluorescence of the fibrinogen-coated microspheres was greater than that of microspheres that had not been incubated with fibrinogen. Product 1 shows lower percentage positive and MFI values than for the Glyⁿ=6 product described in Table 1. Without being bound by theory, we believe that the difference is as a result of the lower fibrinogen concentration used in the production of Product 1.

To determine the effect of fibrinogen concentration on activity of products of the invention, microspheres were prepared using DNTB to link GPRPGGGGGGC (SEQ. ID NO:23) according to the method described above.

To 1 ml of microspheres obtained from step (iii) of method 1 of Example 1, 1 ml of fibrinogen at 1 mg/ml, 0.1 mg/ml or 0.01 mg/ml was added and mixed for 1 hour at room temperature. A zero concentration control was included.

A reference batch control, having fibrinogen covalently linked to the microsphere surface (prepared in the manner discussed above) was also included in the analysis.

The fibrinogen binding to the microspheres was compared, by measuring the percentage absorbance and median fluorescence intensity, as discussed above. Results are shown in Table 3 below.

**Table 3: Fibrinogen binding to products of the invention as defined by claims 1-27 incubated in varying fibrinogen concentrations, or Reference Batch of microspheres**

| **Product Description** | **1 mg/ml** | **0.1 mg/ml** | **0.01 mg/ml** | **0 mg/ml** | **Reference batch** |
|---|---|---|---|---|---|
| **Percent positive** | 71.3±3.18 | 76.2±11.10 | 32.6±5.37 | 0 | 94.7±0.78 |
| **Median fluorescence intensity** | 12.5±5.73 | 4.6±1.14 | 1.24±0.06 | 0 | 22.4±0.49 |

These results show that different amounts of fibrinogen can be bound to the products depending upon the concentration of fibrinogen mixed with the microspheres. This is based on considering the percentage of microspheres which bind anti-fibrinogen antibody, and the fluorescence of the microspheres which is a measure of the amount of fibrinogen bound to each microsphere. As can be seen, 95% of the microspheres in the reference batch are positive for fibrinogen, with a median fluorescence intensity (MFI) of 22.4. In contrast the MFI for fibrinogen binding on the artificial platelets is 12.5 (when incubation occurs at 1 mg/ml), 4.6 (0.1 mg/ml) and 1.24 (0.01 mg/ml).

Absolute numbers of molecules can be determined by reference to calibrated standard fluorescent beads such as provided by Biocytex (for example, the calibration kit for the measurement of platelet glycoprotein expression level and any other human platelet surface molecules, product no. 7011, Biocytex, 140 Ch. Armee d'Afrique, 13010 Marseille, France).

Alternatively the fibrinogen content of the agents can be measured using a modified ELISA assay using an antibody suitable to bind a component in the carrier (for example, where the carrier comprises human albumin, then an anti-human HSA antibody may be used) as the solid-phase capture antibody, and HRP-conjugated rabbit anti-human fibrinogen antibody to detect the fibrinogen. Soluble fibrinogen bound to a plate can be used as a standard.

### Example 3: Assessment of the activity of the products

To determine whether the fibrinogen bound to the peptide on the agent of the invention as defined by claims 1-27 can still be cleaved by thrombin to form fibrin, an agent of the invention can be treated with human thrombin. Cross-linking of the agent of the invention as defined by claims 1-27 via fibrin-fibrin bridging can be tested in a modified aggregation assay, for example, a protocol based on Levi et al, 1999, Nature Medicine, 51, 107-111. The method that we used is reported below.

### 1. Method for assessing the response of products to Thrombin in platelet-free plasma.

The assay was performed using a light transmission aggregometer (we used a PAP4 aggregometer (BioData Corporation)). Thrombin was added to a mixture of plasma (without platelets) and product. The thrombin cleaves the fibrinogen in the plasma and on the microspheres allowing fibrin bridges to form between adjacent microspheres. Aggregation of the microspheres causes an increase in light transmission, which can be recorded as percentage "aggregation" relative to plasma alone.
(i) The count of the products, reference batch or controls was adjusted to 100 x 10³/ml. The microspheres can be counted using a particle counter such as a Flow cytometer (e.g. Beckman Coulter MCL-XL) or Coulter Z2.
(ii) Platelet-free plasma was prepared by centrifuging blood for 30 minutes at 1500 × *g* and collecting the supernatant.
(iii) 350µl platelet-free plasma was placed in an aggregometer cuvette and 50µl of blank microspheres were added. 10, 20 or 40µl of thrombin were added at 3.2 u/ml to each aliquot in the PAP4 aggregometer to achieve a final concentration of 0.08, 0.04 or 0.02 u/ml thrombin. The change in light transmission was measured over a 10 minute period. The thrombin concentration that gave suboptimal light transmission (i.e. minimum clotting with plasma) alone was selected. Typically, this was found to be 20µl.
(iv) 50µl of each of the test preparations or product, reference batch or control microspheres were added to 350µl of platelet-free plasma. The increase in light transmission over 20 minutes in the presence and absence of suboptimal thrombin (determined as described above) was measured. Results are report in Table 4.

**Table 4: Aggregation of Product 1, Product 2 and Reference Batch of microspheres in platelet-free plasma (mean ± sd of 4 experiments)**

| | **Product 1** | **Product 2** | **Reference batch** |
|---|---|---|---|
| **Percentage aggregation in the absence of thrombin** | 4.5 ± 4.1 | 4.75 ± 4.4 | 0.25 ± 0.5 |
| **Percentage aggregation in the presence of thrombin** | 90.0 ± 18.5 | 17.5 ± 14.1 | 20.5 ± 8.7 |

These results demonstrate that Product 1 was aggregated by the action of thrombin on the fibrinogen bound to the surface. The reference batch was aggregated less effectively by thrombin indicating a less favourable configuration of the fibrinogen that was unable to form effective fibin-fibrin cross-linkages.

Product 1 showed aggregation within 5 minutes of addition of thrombin whereas albumin microspheres without fibrinogen on the surface (Product 2) showed lower aggregation (Table 4). Without being bound by theory, Product 2 is thought to show reduced aggregation compared to Product 1 because the assay conditions are thought to allow insufficient time for it to bind fibrinogen from the plasma prior to the addition of thrombin.

We also assessed the response of the products reported in Table 3 above (i.e. produced by incubation with varying concentrations of fibrinogen) to thrombin according to the same method as discussed above. The results are reported in Table 5 below (n=4).

**Table 5: Aggregation to products of the invention as defined by claims 1-27 incubated in varying fibrinogen concentrations and Reference Batch of microspheres in platelet-free plasma (mean ± sd of 4 experiments)**

| **Product Description** | **1 mg/ml** | **0.1 mg/ml** | **0.01 mg/ml** | **0 mg/ml** | **Reference batch** |
|---|---|---|---|---|---|
| **Percentage aggregation (-thrombin)** | 4.75± 4.43 | 3.75± 2.36 | 4.5±4.12 | 4.25±4.93 | 0.25±0.5 |
| **Percentage aggregation (+thrombin)** | 87.25± 21.56 | 90.75± 18.5 | 31.25± 11.81 | 17.5± 14.25 | 20.5±8.74 |

These results show that the response to sub-optimal thrombin of the reference batch is comparable to that seen with the peptide to which no fibrinogen has been bound prior to analysis. The response in the products having fibrinogen linked to the peptide is greatest in the sample made using 0.1 mg/ml fibrinogen (which exhibits an MFI of 4.6).

In addition as a measure of the action of thrombin on the bound fibrinogen the material released can be analysed for fibrinopeptide A, using a commercially available ELISA method supplied by American Diagnostica.

The composition of the platelet substitute can be validated with respect to the pharmacological activity. That the immobilised fibrinogen exhibits the same or similar characteristics as soluble fibrinogen with respect to the interaction with platelets can be demonstrated. Methods are described below which demonstrate that the product will interact preferentially with activated platelets (i.e. only in the presence of an agonist e.g. ADP or thrombin).

### 2. Assessment of activity of candidate preparations of the agent of the invention as defined by claims 1-27 in specific in vitro assays

A number of well-defined, *in vitro* assays can be used to evaluate different aspects of the interaction of the agent of the invention as defined by claims 1-27 with platelets and the haemostatic system, namely platelet aggregation, platelet activation, platelet-dependent thrombus formation and adhesion under conditions of flow, thrombin generation and fibrinolysis.

Fresh platelets are used, obtained locally, from normal volunteers. Blood is collected by clean venepuncture, via a 21 gauge butterfly needle either into an anticoagulant (normally trisodium citrate, or anticoagulant citrate dextrose (ACD), and used as soon as possible, preferably within 15, more preferably 10, minutes of collection to avoid activation of the platelets *in vitro* prior to testing. To study the effect of the agent of the invention as defined by claims 1-27 in blood with low platelet counts the platelets are depleted by centrifugation and the blood reconstituted in autologous plasma, as described below.

To artificially deplete platelets in normal blood, the blood can be centrifuged at 150 × *g* for 20 minutes at room temperature. The platelet rich plasma is removed, and diluted with autologous plasma prepared from a separate tube of blood that has been centrifuged at 1800 × *g* for 30 minutes. The platelet poor plasma is then added back to the platelet rich plasma and carefully mixed. This platelet-depleted plasma can also be added back to the autologous red cells to prepare platelet-depleted whole blood. A reduction of approximately 65 to 95% of the original platelet count is achieved by this method. Platelet counts are measured in all samples using a blood cell counter such as the AC^{T}Diff; (Beckman-Coulter).

The methods that we used are described below.

### 3. Methods for preparing "thrombocytopenic blood" in the laboratory

### 3.1 Preparation of Platelet-Depleted Whole Blood

(i) Two tubes of blood were collected into tri-sodium citrate anticoagulant, by clean venepuncture via a 21 gauge butterfly needle. The blood was processed within 10 minutes of collection, to minimise platelet activation *in vitro.*
(ii) One tube of blood was centrifuged at 150 × *g* for 20 minutes at 20°C to prepare platelet-rich plasma (PRP).
(iii) A second tube of blood was centrifuged at 1500x g for 30 minutes at 20°C to prepare platelet-poor plasma (PPP).
(iv) The PRP was removed from the blood cell fraction and replaced by the PPP. By this means the platelet count was reduced by approximately 75% to achieve a count of approximately 50x10⁶ platelets/ml.
(v) Larger volumes of platelet-depleted blood can be prepared by using several blood tubes.

### 3.2 Preparation of Platelet-Depleted Plasma

(i) Two tubes of blood were collected into tri-sodium citrate anticoagulant, by clean venepuncture via a 21 gauge butterfly needle. The blood was processed within 10 minutes of collection, to minimise platelet activation *in vitro.*
(ii) One tube of blood was centrifuged at 150 × g for 20 minutes at 20°C to prepare platelet rich plasma (PRP).
(iii) A second tube of blood was centrifuged at 1500 × g for 30 minutes at 20°C to prepare platelet poor plasma (PPP).
(iv) The PRP was removed from the blood cell fraction and mixed with the PPP to achieve a platelet count of between 10 and 50x10⁶ platelets/ml.
(v) Larger volumes of platelet depleted plasma can be prepared by using several blood tubes.

With both methods the residual platelets in the blood or plasma can be tested to ensure that the platelets have not been activated during preparation, for example by measuring the binding of plasma fibrinogen to activated GPIIb-IIIa according to the methods known in the art, such as those described by Janes et al, Thrombosis & Haemostasis, 1993, 70, 659-666.

### 4. Assessment of agents of the invention on Platelet Aggregation

This method can be used to show that agents of the invention as defined by claims 1-27 enhance platelet aggregation in the presence of an agonist only. A Beckman Coulter MCL-XL flow cytometer, and AC^{T}Diff cell counter, and a PAP4 Bio/Data Corporation platelet aggregometer are available for measuring platelet aggregation in whole blood, in platelet-rich plasma (PRP), platelet-depleted whole blood or platelet-depleted plasma. Either spontaneous aggregation (in the absence of agonist) or aggregation in the presence of an agonist such as ADP (adenosine diphosphate), TRAP (thrombin receptor activation peptide) and CRP (collagen related peptide) can be determined, and compared to results obtained with and without test microspheres, for example in a stirred system.

For example, the agent of the invention as defined by claims 1-27 can be added to either platelet rich plasma, or to platelet-depleted plasma, and incubated at 37° C with stirring. Rate and % aggregation can be measured in by measuring the increase in light transmission, in an aggregometer such as the PAP4.

An exemplary method for studying the interaction of artificial platelets with platelets in the presence and absence of an agonist is described below.

### 4.1 Method for measuring aggregation by counting in a flow cytometer

Flow cytometry can be used to count the number of residual single platelets, and thereby to calculate the percentage of platelets that have bound to artificial platelets. The sample is analysed by the forward and side scatter and an electronic gate is set around the population of single platelets. To do this a sample of platelet-rich plasma is used that contains EDTA to prevent platelet-platelet aggregation occurring. The platelets are labelled with a fluorescently-conjugated antibody such as a CD42b monoclonal that binds to the GPIb receptor on all platelets. In this way the number of CD42b-positive events in the platelet gate give an accurate measure of the number of platelets, especially when the sample contains artificial platelets, which do not bind the CD42b antibody.

As aggregation occurs the number of platelets within the gate declines. The artificial platelets should not induce aggregation of non-activated ("resting") platelets. If, however, the platelets are activated, for example by ADP they should aggregate with the artificial platelets. In this assay a sub-optimal concentration of ADP is used to distinguish the effects of the artificial platelets in platelet-deficient plasma.
(i) To set the platelet gate, whole blood that had been collected into EDTA was used. 5µl of EDTA anticoagulated blood was added to 50µl Hepes-buffered saline and analysed by forward and side scatter in a flow cytometer (e.g. Beckman Coulter MCL-XL). A gate was set around the platelet population so that >98% of the single platelets are contained within the platelet gate.
(ii) 400µl of platelet-depleted plasma, with a platelet count of 10-60 x 10⁶/ml, was mixed with 20µl of control microspheres (blank capsules as disclosed in Example 1, Method 1(i)) at 10mg albumin/ml and ADP was added at final concentrations of between 1 and 4x10⁻⁵ M. The sample was stirred at 900 rpm at 37°C in an aggregometer cuvette for up 10 minutes and aggregation was measured as the change in light transmission (as described in section 4.2 below). The ADP concentration that gave >0% and <20% aggregation was used for the subsequent analysis of the interaction of the product with platelet-depleted plasma. We found that an ADP concentration of 2x10⁻⁵ M was suitable.
(iii) To test the products, 400µl of platelet-depleted plasma, with a platelet count of 10-60 x 10⁶/ml, was mixed with 20µl of test microspheres at 10mg albumin/ml and ADP at a concentration determined as described above. The sample was mixed at 900 rpm for up to 10 minutes. At appropriate times 5µl aliquots were removed for flow cytometric analysis as follows.
(iv) For the analysis 5µl of test sample was added to 50µl Hepes-buffered saline containing an appropriate concentration of a fluorescently-conjugated antibody to an antigen present on all platelets. We used 1µl of an RPE-conjugated CD42b monoclonal antibody (we used antibody 555473 provided by BD Pharmingen). The sample was incubated for 20 minutes at room temperature (typically 20°C) then diluted with 0.5ml formyl saline (0.2% formalin in 0.9% NaCl).
(v) The diluted sample was then analysed by forward and side scatter in a flow cytometer (e.g. Beckman Coulter MCL-XL) using the gate set around the platelet population in the control sample (see 4.1.i), allowing a fixed volume of sample (e.g. 20µl) to pass through the cytometer. Particles enclosed in the platelet gate were analysed for fluorescence to identify the number of single platelets within the gate. A negative control for fluorescence was set using an isotype control for the antiplatelet antibody conjugated to the same fluorochrome (e.g. RPE-mouse IgG_{1κ} from BD Pharmingen).
(vi) Data were recorded as the percentage loss of single platelets (i.e. CD42b-positive particles) from the platelet gate. Results are shown in Table 6 below.

**Table 6: Aggregation of Product 1. Product 2 and Reference Batch of microspheres in platelet-depleted plasma (mean ± sd of 3 experiments)**

| | **Platelet depleted plasma** | **Product 1** | **Product 2** | **Reference batch** |
|---|---|---|---|---|
| **Percentage aggregation in the absence of ADP** | 5.0 ± 1.8 | 6.6 ± 1.8 | 17.8 ± 3.5 | 52.9 ± 3.3 |
| **Percentage aggregation in the presence of ADP** | 29.3 ± 7.8 | 65.7 ± 9.2 | 57.3 ± 9.3 | 50.8 ± 19.8 |

These results show that Product 1 provides a significantly greater degree of aggregation of the activated platelets in the platelet-depleted plasma than is seen with the platelets alone, but no increase in aggregation of inactive platelets, in the absence of ADP. By contrast, the reference batch, prepared essentially as taught in WO 98/17319 caused similar and significant levels of aggregation with both resting (inactive) and activated platelets.

The same method can be used to illustrate that peptide-coupled microspheres can also aggregate platelets utilising the fibrinogen present in the plasma. In this example peptide-coupled microspheres caused only 17.8±3.5% aggregation in the absence of ADP but 57.3±9.3% aggregation when the platelets had been activated with ADP.

We also assessed the effect of the products reported in Table 3 above (i.e. produced by incubation with varying concentrations of fibrinogen) on aggregation of platelet-deficient plasma in the presence and absence of ADP, according to the same method as discussed above. The results are reported in Table 7 below (n=3).

**Table 7: Aggregation to products of the invention incubated in varying fibrinogen concentrations and Reference Batch of microspheres in platelet-depleted plasma (PDP)**

| **Product Description** | **PDP** | **1 mg/ml** | **0.1 mg/ml** | **0.01 mg/ml** | **0 mg/ml** | **Reference batch** |
|---|---|---|---|---|---|---|
| **Percentage aggregation (-ADP)** | 5.09± 1.85 | 13.3± 5.76 | 5.1± 1.81 | 9.3± 8.06 | 17.9± 3.45 | 51.9 ± 3.31 |
| **Percentage aggregation (+ADP)** | 29.3± 7.88 | 60.3± 4.75 | 65.6± 9.17 | 51.9± 7.44 | 57.6± 7.64 | 50.8± 19.86 |

These results show that the highest result, 65.6% aggregation, was obtained with the sample exhibiting an MFI of 4.6. In contrast the reference batch only induced 50% aggregation in activated platelets, this result also being observed in the absence of ADP (i.e. in inactive platelets also).

From these data we conclude that there may not be a simple linear relationship between the amount of fibrinogen linked to a microsphere and the activity of the microsphere. In addition, in spite of the fact that the reference batch product exhibits a high fibrinogen content, it is not as active as the artificial platelets of the present invention.

If agent of the invention as defined by claims 1-27 is added to whole blood, rate or % aggregation can also be measured by counting residual platelets, for example in a flow cytometer.

### 4.2 Method for measuring aggregation by light transmission using the PAP-4 platelet aggregometer

In this system the product was mixed with platelet-depleted plasma in a stirred system at 9000 rpm and aggregation was measured by a change in light transmission.
(i) 400µl of platelet-depleted plasma was mixed with 20µl of blank microspheres at 10mg albumin/ml.
(ii) ADP was added at concentrations between 1 and 4 x 10⁻⁵M
(iii) Aggregation was measured over 20 minutes and recorded as the percentage of maximum using autologous plasma to set the value for 100% aggregation.

The results of testing the aggregation of Product 1, Product 2 and the Reference Batch of microspheres in platelet-depleted plasma, using the method above, as measured by aggregometry, showed that the Reference Batch caused high aggregation in the absence of ADP whereas Products 1 and 2 showed only low levels of aggregation in the absence of ADP. Moreover, Product 1 showed significantly higher levels of aggregation when ADP was added.

Typically, agents of the invention, as defined by claims 1-27 when tested by these methods, will show a greater increase in the aggregation of active platelets than inactivated platelets as defined above. This is illustrated in Tables 6 and 7 for Product 1.

### 5. Flow cytometric analysis of the interaction of the agent of the invention with platelets.

A Coulter Epics XL MCL Flow Cytometer can be used to examine of the stoichiometry of interactions between platelets and the agent of the invention, and to assay whether the agent of the invention causes platelet activation, as demonstrated by a change in surface antigens (e.g. P selectin) in the absence of exogenous stimulation. P selectin is a marker of platelet degranulation and therefore platelet activation.

The agent of the invention as defined by claims 1-27 is added to whole blood and mixed in a controlled manner, with and without exogenous agonists such as ADP, TRAP, and CRP. Aliquots of these mixtures are diluted in HEPES buffered saline containing fluorescently conjugated Mabs and incubated to allow antibody binding. For example R-Phycoerythrin(RPE) conjugated mouse monoclonal to human GPIb (Cy5RPE) available from BD Biosciences, Oxford, UK and a FITC-labelled polyclonal antibody to human serum albumin (Autogen Bioclear Ltd) can be used to identify the platelets and agents of the invention respectively and to measure interactions between platelets and the agent of the invention with and without agonist stimulation. In addition FITC-conjugated Mabs to markers of platelet activation can be used to show whether platelets either free or bound to agent of the invention are activated. Activation of GPIIb-IIIa complex (a prerequisite of platelet aggregation) can be measured using a Mab PAC-1 which recognises an epitope on the GPIIb/IIIa complex of activated platelets at or near the platelet fibrinogen receptor (Becton Dickenson Immunocytometry Systems). Alternatively, a Mab specific for platelet P selectin can be used as a marker of platelet degranulation (Serotec).

Following incubation with the relevant antibody fluorescence is measured on the Flow Cytometer. Typically, agents of the invention will cause relatively little activation of platelets as measured by PAC-1 Mab or P-selectin binding, as described above. In one embodiment, the term. "relatively little" means that agents of the invention as defined by claims 1-27 cause less platelet activation than prior art platelet substitutes as defined above.

### 6. Study of the interaction of the agent of the invention as defined by claims 1-27 with platelets under conditions of high shear rate flow

A Parallel Plate Perfusion Chamber can be used to study the interaction of the agent of the invention as defined by claims 1-27 with platelets, under conditions of flow, using variable rates of shear.

As a control, surface coverage is observed when a normal blood sample is perfused at different shear rates over sub-endothelial matrix or a collagen substrate (Levi et al, 1999, Nature Medicine, 51, 107-111). Then surface coverage is observed using the same protocol but with a platelet-depleted sample prepared as described above. Surface coverage should be reduced in the platelet-depleted sample, compared to the normal blood sample. Agents of the invention will preferably be able to increase surface coverage when added to the platelet-depleted sample.

When the agents of the invention as defined by claims 1-27 are added to the platelet-depleted sample in sufficient numbers to stoichiometrically replace the depleted platelets (i.e. platelet-depleted samples prepared as described above are reduced to a platelet count of about 25% of the undepleted sample: for the purposes of this test, agents of the invention are added in sufficient numbers to return the total number of remaining platelets plus agents of the invention to substantially 100% of the original platelet count in the undepleted sample), then it is particularly preferred if the agent of the invention as defined by claims 1-27 is able to increase surface coverage in the platelet-poor sample to a level that is substantially equivalent to (e.g. 50, 60, 70, 80, 90 or 100%), or even higher than, the level of surface coverage observed for normal blood under the same flow conditions.

The extent of surface coverage by deposition of platelets or a combination of platelets and agents of the invention as defined by claims 1-27 can be determined microscopically.

### Example 4: Evaluation of FLPs in vivo models:

### 1. Dose related haemostatic activity in a thrombocytopenic rabbit model

Male New Zealand white rabbits 2.5-3.0kg (approximately 4 months old) are obtained from a reputable supplier. Groups of six rabbits are rendered thrombocytopenic using two doses of busulphan, 12 and 9 days respectively, prior to the study day. The dose of busulphan is varied according to the severity of thrombocytopenia required, e.g. two doses of 20mg/kg will generally reduce the platelet count to between 10-20 x10⁹/l, whereas two doses of 25mg/kg will reduce the platelet count to less than 10x10⁹/l. In addition to a reduction in platelet count, busulphan dosing is associated with depletion of white cells, but only a minor reduction in haematocrit and no overt toxicity. No anaesthetic is required for this procedure.

Human platelet concentrates are used as a positive control for these studies. This requires only one platelet concentrate per group of animals. It has been shown previously that human platelets circulate for only approximately 5 minutes in the rabbit, due to uptake by the reticulo-endothelial system. Therefore in these experiments macrophage function is inhibited by dosing the rabbits with ethyl palmitate 24 hours before the study day. For consistency, treatment with ethyl palmitate is used for all groups of animals.

On the study day the test agent is infused intravenously into an ear vein. Efficacy is assessed by measurement of bleeding time, which is performed using a standard (Simplate) incision in the ear.

Variability in the bleeding time is controlled, as far as possible, by ensuring that the animals are quiet and warm and at an even temperature (Roskam, 1993, Comptes Rendus des Seances de la Societé de Biologie, 114, 166-169), and the number of blood samples minimised. Blajchman & Lee, 1997, Transfusion Medical Reviews, 11, 99-105. Bleeding times are measured immediately prior to administration of the test dose and at four time points, up to 24 hours after dosing. Bleeding times in excess of 20 minutes are stopped by applying pressure to the wound. Animals are sacrificed at the completion of the study.

Dose related activity of the platelet substitute as defined by reduction in bleeding time is compared to the activity of human platelets (dose/kg basis). HSA microparticles of the same size but with no coupled peptide are used as the negative control. A comparison of the duration of effect over the 24 hour period of the study is also made.

In a thrombocytopenic rabbit, a bleeding time of about 20 minutes is typical. Platelet substitutes of the disclosure will generally be able to reduce the bleeding time to less than 10 minutes in a minimum of three, and preferably all six, of the test rabbits in the group of six.

### 2. Assessment of the agent of the invention's as defined by claims 1-27 thrombogenicity in a rabbit Wessler model

The potential thrombogenicity of the candidate FLP preparation is assessed in the Wessler model, essentially as described by Wessler et al, 1959, Journal of Applied Physiology, 14, 943-946, but including controls appropriate to a platelet substitute. Controls are defined by consideration of data obtained from *in vitro* methods.

Male New Zealand White rabbits, body weight 2.5kg-3.0kg (approx 4 months) are obtained from an approved supplier and groups of six rabbits anaesthetised. Segments of the right and left jugular veins are exposed and detached from the surrounding tissue. The test preparations are administered through an ear vein and following a period of circulation of 3 minutes the segments of the jugular veins are ligatured and left *in situ* for a further 10 minutes. The segments are carefully excised, and the lumen exposed. The vessel is examined for the presence of developed thrombi, which is scored visually.

Platelet substitutes of the disclosure should not produce thrombi at doses which are 5-fold, preferably 10-fold, higher than the dose associated with optimal reduction in bleeding time.

A suitable dose may be between 1×10⁸ to 2×10¹⁰ product particles per kg of patient body weight. For example, the dose (when expressed a number of product particles per kg body weight) may be about 2×10⁸, 3×10⁸, 4×10⁸, 5×10⁸, 6×10⁸, 7×10⁸, 8×10⁸, 9×10⁸, 1×10⁹, 2×10⁹, 3×10⁹, 4×10⁹, 5×10⁹, 6x10⁹, 7×10⁹, 8×10⁹, 9×10⁹, 1×10¹⁰ or 2×10¹⁰.

A suitable dose may also be expressed as milligrams of total protein per kg patient body weight. On this basis a suitable dose may be between 5-200 mg/kg. For example, the dose may be about 5, 10, 15, 20, 30, 40, 50, 60, 70, 80, 90, 150 or 200 mg/kg.

An ideal dose has a safety margin of at least two-fold, preferably about 10-fold. In other words, the ideal dose is effective but remains safe even when increased by two-fold or about 10-fold. A safe dose does not form a clot using the Wessler test as described above.

## Claims

1. An injectable pharmaceutical product comprising an agent, the agent comprising an insoluble carrier to which is bound a peptide having affinity for fibrinogen, the peptide being capable of non-covalently binding fibrinogen such that the agent binds via the non-covalently bound fibrinogen to activated platelets in preference to inactive platelets, and wherein the peptide is not fibrinogen.

2. A product according to Claim 1 in which, if the product is introduced intravenously, the peptide non-covalently binds fibrinogen such that the non-covalently bound fibrinogen will preferentially become involved in formation of a blood clot at the site of a wound where platelets are already activated.

3. A product according to Claim 1 or 2 wherein the peptide comprises a fibrinogen-binding sequence obtained from the platelet membrane glycoprotein GPllb or GPllla, such as the sequence TDVNGDGRHDL (SEQ. ID NO: 6) or a variant of such a sequence.

4. A product according to any one of the preceding claims wherein the peptide comprises TDVNGDGRHDL (SEQ. ID NO: 6).

5. A product according to any one of the preceding claims wherein the peptide comprises the sequence of Gly-(Pro/His)-Arg-Xaa (SEQ. ID NO: 24) at the amino terminus, wherein Xaa is any amino acid.

6. A product according to Claim 5 wherein Xaa is Pro, Sar, Gly or Val (EQ ID NO: 25).

7. A product according to any one of the preceding claims wherein the peptide has from 4 to 200 amino acids.

8. A product according to any one of the preceding claims wherein the carrier has a size suitable to ensure transmission of the agent through the lung capillary bed.

9. A product according to any one of the preceding claims wherein the carrier is a microparticle.

10. A product according to Claim 11 wherein the microparticle is a protein microparticle, such as an albumin microparticle.

11. A product according to any one of Claims 8 to 10 wherein the product comprises a population of carriers of which less than 2% are in excess of 6 µm as a maximum dimension.

12. A product according to any one of Claims 8 to 11 wherein the majority of carriers are from 2 to 4 µm as a maximum dimension.

13. A product according to any one of the preceding claims wherein the peptide is bound to the carrier by a covalent bond.

14. A product according to Claim 13 wherein the peptide comprises a cysteine and is bound to the carrier by linking the -SH group of the cysteine to a thiol reactive group on the carrier.

15. A product according to any one of the preceding claims wherein the product additionally comprises fibrinogen, or a variant or fragment thereof, having an inducible platelet-aggregating activity, non-covalently bound to the said peptide.

16. An injectable pharmaceutical product having an inducible platelet-aggregating activity comprising an insoluble carrier to which fibrinogen, or a variant or fragment thereof, is non-covalently bound, via a peptide having an affinity for fibrinogen, in a configuration such that the fibrinogen binds to activated platelets in preference to inactive platelets.

17. A product according to Claim 16 which, when introduced intravenously, will only become involved significantly in formation of a blood clot at the site of a wound where platelets are already activated.

18. A method for preparing a product as defined in any one of Claims 15 to 17, comprising providing a product according to any one of Claims 1 to 14 and mixing with fibrinogen, or a variant of fragment thereof and optionally further comprising one or more of the following steps -
(a) removing unbound fibrinogen;
(b) formulating the product with a pharmaceutically acceptable carrier or diluent;
(c) diluting the product to provide a pharmaceutically acceptable unit dose; and
(d) sterilising the product, or ensuring product sterility throughout steps (a) to (c).

19. A product as defined in any one of Claims 1 to 17 for use in medicine.

20. A product as defined in any one of Claims 1 to 17 for the treatment of a patient with thrombocytopenia.

21. Use of a product as defined in any one of Claims 1 to 17 in the manufacture of a medicament for the treatment of a patient with thrombocytopenia.

22. Use according to any one of Claims 19 to 21 wherein the patient has a platelet count below 400x10⁹/l, preferably below 150x10⁹/l.

23. Use according to Claim 22 wherein the platelet count is below 10x 10⁹/l.

24. Use according to any one of Claims 19 to 23 wherein the patient has a failure in platelet production from the bone marrow.

25. Use according to Claim 24 wherein the failure in platelet production from the bone marrow is caused by a blood cancer, or cytotoxic chemotherapy or radiotherapy.

26. Use according to Claim 19 wherein the patient has an inherited or drug-induced disorders in platelet number or function.

27. Use according to Claim 19 wherein the patient's platelets have been mechanically damaged.

## Patentansprüche

1. Injizierbares pharmazeutisches Produkt, das ein Mittel beinhaltet, wobei das Mittel einen unlöslichen Träger beinhaltet, an den ein Peptid mit Affinität zu Fibrinogen gebunden ist, wobei das Peptid in der Lage ist, Fibrinogen nicht kovalent zu binden, so dass sich das Mittel über das nicht kovalent gebundene Fibrinogen bevorzugter an aktivierte Plättchen als an inaktive Plättchen bindet, und wobei das Peptid nicht Fibrinogen ist.

2. Produkt nach Anspruch 1, in dem, wenn das Produkt intravenös eingeführt wird, das Peptid Fibrinogen nicht kovalent bindet, so dass das nicht kovalent gebundene Fibrinogen vorzugsweise an der Stelle einer Wunde, wo Plättchen bereits aktiviert sind, in der Bildung eines Blutgerinnsels involviert wird.

3. Produkt nach Anspruch 1 oder 2, wobei das Peptid eine Fibrinogen-Bindungssequenz beinhaltet, die vom Plättchenmembran-Glykoprotein GPIIb oder GPIIIa erhalten wird, wie die Sequenz TDVNGDGRHDL (SEQ ID Nr. 6) oder eine Variante einer solchen Sequenz.

4. Produkt nach einem der vorherigen Ansprüche, wobei das Peptid TDVNGDGRHDL (SEQ ID Nr. 6) beinhaltet.

5. Produkt nach einem der vorherigen Ansprüche, wobei das Peptid die Sequenz von Gly-(Pro/His)-Arg-Xaa (SEQ ID Nr. 24) am Aminoterminus beinhaltet, wobei Xaa eine beliebige Aminosäure ist.

6. Produkt nach Anspruch 5, wobei Xaa Pro, Sar, Gly oder Val (SEQ ID Nr. 25) ist.

7. Produkt nach einem der vorherigen Ansprüche, wobei das Peptid 4 bis 200 Aminosäuren hat.

8. Produkt nach einem der vorherigen Ansprüche, wobei der Träger eine Größe hat, die geeignet ist, eine Übertragung des Mittels durch das Lungenkapillarbett zu ermöglichen.

9. Produkt nach einem der vorherigen Ansprüche, wobei der Träger ein Mikropartikel ist.

10. Produkt nach Anspruch 11, wobei der Mikropartikel ein Proteinmikropartikel wie ein Albuminmikropartikel ist.

11. Produkt nach einem der Ansprüche 8 bis 10, wobei das Produkt eine Population von Trägern beinhaltet, von denen weniger als 2 % eine maximale Abmessung von mehr als 6 µm haben.

12. Produkt nach einem der Ansprüche 8 bis 11, wobei die Mehrzahl der Träger eine maximale Abmessung von 2 bis 4 µm hat.

13. Produkt nach einem der vorherigen Ansprüche, wobei das Peptid über eine kovalente Bindung an den Träger gebunden ist.

14. Produkt nach Anspruch 13, wobei das Peptid ein Cystein beinhaltet und an den Träger durch Verknüpfen der -SH Gruppe des Cysteins mit einer Thiol-reaktiven Gruppe auf dem Träger gebunden ist.

15. Produkt nach einem der vorherigen Ansprüche, wobei das Produkt zusätzlich Fibrinogen oder eine Variante oder ein Fragment davon mit einer induzierbaren Plättchenaggregationsaktivität beinhaltet, das/die nicht kovalent an das genannte Peptid gebunden ist.

16. Injizierbares pharmazeutisches Produkt mit einer induzierbaren Plättchenaggregationsaktivität, das einen unlöslichen Träger beinhaltet, an den Fibrinogen oder eine Variante oder ein Fragment davon über ein Peptid nicht kovalent gebunden ist, das eine Affinität zu Fibrinogen hat, in einer solchen Konfiguration, dass sich das Fibrinogen bevorzugter an aktivierte Plättchen als an inaktive Plättchen bindet.

17. Produkt nach Anspruch 16, das, wenn es intravenös eingeführt wird, nur an der Stelle einer Wunde, wo Plättchen bereits aktiviert sind, wesentlich in der Bildung eines Blutgerinnsels involviert wird.

18. Verfahren zur Herstellung eines Produkts wie in einem der Ansprüche 15 bis 17 definiert, das das Bereitstellen eines Produkts nach einem der Ansprüche 1 bis 14 und Mischen mit Fibrinogen oder einer Variante oder einem Fragment davon und optional ferner einen oder mehrere der folgenden Schritte beinhaltet:
(a) Entfernen von ungebundenem Fibrinogen;
(b) Formulieren des Produkts mit einem pharmazeutisch akzeptablen Träger oder Verdünnungsmittel;
(c) Verdünnen des Produkts, um eine pharmazeutisch akzeptable Einheitsdosis zu erhalten; und
(d) Sterilisieren des Produkts oder Gewährleisten der Produktsterilität in den Schritten (a) bis (c).

19. Produkt wie in einem der Ansprüche 1 bis 17 definiert zur Verwendung in der Medizin.

20. Produkt wie in einem der Ansprüche 1 bis 17 definiert zur Behandlung eines Patienten mit Thrombozytopenie.

21. Verwendung eines Produkts wie in einem der Ansprüche 1 bis 17 definiert zur Herstellung eines Medikaments für die Behandlung eines Patienten mit Thrombozytopenie.

22. Verwendung nach einem der Ansprüche 19 bis 21, wobei der Patient eine Plättchenzahl unter 400 x 10⁹/l, vorzugsweise unter 150 x 10⁹/l hat.

23. Verwendung nach Anspruch 22, wobei die Plättchenzahl unter 10 x 10⁹/l ist.

24. Verwendung nach einem der Ansprüche 19 bis 23, wobei der Patient eine Fehlfunktion der Plättchenproduktion vom Knochenmark hat.

25. Verwendung nach Anspruch 24, wobei die Fehlfunktion der Plättchenproduktion vom Knochenmark durch Blutkrebs oder zytotoxische Chemotherapie oder Radiotherapie verursacht wird.

26. Verwendung nach Anspruch 19, wobei der Patient eine geerbte oder arzneimittelinduzierte Störung der Plättchenanzahl oder -funktion hat.

27. Verwendung nach Anspruch 19, wobei die Plättchen des Patienten mechanisch beschädigt wurden.

## Revendications

1. Produit pharmaceutique injectable comprenant un agent, l'agent comprenant un vecteur insoluble auquel est lié un peptide ayant une affinité pour le fibrinogène, le peptide étant capable de lier de façon non covalente le fibrinogène de sorte que l'agent se lie via le fibrinogène lié de façon non covalente à des plaquettes activées plutôt qu'à des plaquettes inactives, et dans lequel le peptide n'est pas le fibrinogène.

2. Produit selon la revendication 1, dans lequel, si le produit est introduit par voie intraveineuse, le peptide lie de façon non covalente le fibrinogène de sorte que le fibrinogène lié de façon non covalente sera de préférence impliqué dans la formation d'un caillot sanguin au niveau du site d'une blessure où des plaquettes sont déjà activées.

3. Produit selon la revendication 1 ou 2, dans lequel le peptide comprend une séquence de liaison du fibrinogène obtenue à partir de la glycoprotéine de membrane de plaquette GPIIb ou GPIIIa, telle que la séquence TDVNGDGRHDL (SEQ ID NO : 6) ou une variante d'une telle séquence.

4. Produit selon l'une quelconque des revendications précédentes, dans lequel le peptide comprend TDVNGDGRHDL (SEQ ID NO : 6).

5. Produit selon l'une quelconque des revendications précédentes, dans lequel le peptide comprend la séquence de Gly-(Pro/His)-Arg-Xaa (SEQ ID NO : 24) au niveau de l'extrémité amino-terminale, dans laquelle Xaa est un acide aminé quelconque.

6. Produit selon la revendication 5, dans lequel Xaa est Pro, Sar, Gly ou Val (SEQ ID NO : 25).

7. Produit selon l'une quelconque des revendications précédentes, dans lequel le peptide a de 4 à 200 acides aminés.

8. Produit selon l'une quelconque des revendications précédentes, dans lequel le vecteur a une taille adaptée pour assurer la transmission de l'agent à travers le lit capillaire de poumon.

9. Produit selon l'une quelconque des revendications précédentes, dans lequel le vecteur est une microparticule.

10. Produit selon la revendication 11, dans lequel la microparticule est une microparticule de protéine, telle qu'une microparticule d'albumine.

11. Produit selon l'une quelconque des revendications 8 à 10, dans lequel le produit comprend une population de vecteurs dont moins de 2 % ont une dimension maximale supérieure à 6 µm.

12. Produit selon l'une quelconque des revendications 8 à 11, dans lequel la majorité des vecteurs ont une dimension maximale de 2 à 4 µm.

13. Produit selon l'une quelconque des revendications précédentes, dans lequel le peptide est lié au vecteur par une liaison covalente.

14. Produit selon la revendication 13, dans lequel le peptide comprend une cystéine et est lié au vecteur par liaison du groupe -SH de la cystéine à un groupe réactif au thiol sur le vecteur.

15. Produit selon l'une quelconque des revendications précédentes, dans lequel le produit comprend en outre du fibrinogène, ou une variante ou un fragment de celui-ci, ayant une activité d'agrégation de plaquettes inductible, lié de façon non covalente audit peptide.

16. Produit pharmaceutique injectable ayant une activité d'agrégation de plaquettes inductible comprenant un vecteur insoluble auquel du fibrinogène, une variante ou un fragment de celui-ci, est lié de façon non covalente, via un peptide ayant une affinité pour le fibrinogène, dans une configuration telle que le fibrinogène se lie aux plaquettes activées plutôt qu'aux plaquettes inactives.

17. Produit selon la revendication 16 qui, lorsqu'il est introduit par voie intraveineuse, est impliqué significativement uniquement dans la formation d'un caillot sanguin au niveau du site d'une plaie où les plaquettes sont déjà activées.

18. Procédé de préparation d'un produit tel que défini dans l'une quelconque des revendications 15 à 17, comprenant la fourniture d'un produit selon l'une quelconque des revendications 1 à 14 et le mélange avec du fibrinogène, ou une variante ou un fragment de celui-ci et comprenant en outre facultativement une ou plusieurs des étapes suivantes :
(a) élimination du fibrinogène non lié ;
(b) formulation du produit avec un vecteur ou diluant pharmaceutiquement acceptable ;
(c) dilution du produit pour fournir une dose unitaire pharmaceutiquement acceptable ; et
(d) stérilisation du produit, ou garantie de la stérilité du produit tout au long des étapes (a) à (c).

19. Produit tel que défini selon l'une quelconque des revendications 1 à 17, pour une utilisation en médecine.

20. Produit tel que défini selon l'une quelconque des revendications 1 à 17, pour le traitement d'un patient atteint de thrombocytopénie.

21. Utilisation d'un produit tel que défini dans l'une quelconque des revendications 1 à 17 dans la fabrication d'un médicament destiné au traitement d'un patient atteint de thrombocytopénie.

22. Utilisation selon l'une quelconque des revendications 19 à 21, dans laquelle le patient a une numération plaquettaire inférieure à 400 x 10⁹/L, de préférence inférieure à 150 x 10⁹/L,

23. Utilisation selon la revendication 22, dans laquelle la numération plaquettaire est inférieure à 10 x 10⁹/L.

24. Utilisation selon l'une quelconque des revendications 19 à 23, dans laquelle le patient est atteint d'une insuffisance de production plaquettaire provenant de la moelle osseuse.

25. Utilisation selon la revendication 24, dans laquelle l'insuffisance de production plaquettaire provenant de la moelle osseuse est provoquée par un cancer du sang, ou une chimiothérapie ou une radiothérapie cytotoxique.

26. Utilisation selon la revendication 19, dans laquelle le patient souffre de troubles héréditaires ou induits par les médicaments de la numération ou de la fonction plaquettaire.

27. Utilisation selon la revendication 19, dans laquelle les plaquettes du patient ont été endommagées mécaniquement.
